# EUROPEAN PATENT SPECIFICATION

(11) **EP 3 180 616 B1**
(45) Date of publication and mention of the grant of the patent: **26.02.2020**
(21) Application number: 15753979.2
(22) Date of filing: 13.08.2015
(51) Int. Cl.: G01N 33/543, G01N 33/569, G01N 33/58, G01N 33/558

(54) **METHOD AND KIT FOR THE DETECTION OF MYCOBACTERIA**
VERFAHREN UND KIT ZUM NACHWEIS VON MYKOBAKTERIEN
MÉTHODE ET KIT POUR LA DÉTECTION DES MYCOBACTÉRIES

(30) Priority: 13.08.2014 GB 201414369
(43) Date of publication of application: 21.06.2017
(73) Proprietor: Diagnostig Ltd, Wales LL57 2UW (GB)
(72) Inventor: BAIRD, Mark Stephen, Bangor Gwynedd LL57 2DG (GB); GWENIN, Christopher David, Bangor Gwynedd LL57 2DG (GB); GWENIN, Vanessa Valerie, Bangor Gwynedd LL57 2DG (GB); HACKING, Joanne Louise, Darwen Lancashire BB3 3JX (GB); PITTS, Mark, Caernarfon Gwynedd LL55 4BA (GB)
(74) Representative: Appleyard Lees IP LLP
(86) International application number: PCT/GB2015/052338
(87) International publication number: WO 2016/024116

(56) References cited:
- WO-A1-2012/131394
- WO-A1-2012/153111
- WO-A2-2010/086667
- WO-A2-2012/007903
- JP-A- H07 248 329
- US-A1- 2003 143 652
- US-A1- 2003 219 833
- US-A1- 2007 054 335

## Description

The present invention relates to a kit and method for detecting an antibody in a sample. In particular the invention provides a kit and method for determining whether or not an individual is infected with a mycobacterial disease.

Pathogenic and non-pathogenic mycobacteria are very widespread in the environment and their rapid detection and distinction represents an important public health target.

For example, tuberculosis is a serious and often fatal disease which affects humans and other animals and is caused by infection with mycobacteria. Infection with *Mycobacterium tuberculosis* is very common and it is estimated that up to a third of the world's population is infected with the bacterium. Most of those infected will never develop the active disease but because it is often fatal if left untreated, early diagnosis of the disease is essential. Methods of detecting *M*. *tuberculosis* are known but these existing methods have a number of disadvantages. It can often take a long time for the results of a test to be known, the equipment needed is expensive or difficult to use, the results are not always reliable and some methods are not able to distinguish between active and latent tuberculosis. A number of serodiagnostic assays have been developed for the diagnosis of tuberculosis but none of these have been assessed as reaching the standards required by the World Health Organisation.

Other mycobacterial infections can also be particularly problematic for individuals suffering from other medical conditions, such as *Mycobacterium abscessus* in cystic fibrosis patients.

Infection with tuberculosis is also common in cattle and bovine tuberculosis is recognised as a serious problem. Another disease common in cattle is Johne's disease which is caused by infection with another mycobacteria, for example *Mycobacterium avium paratuberculosis.*

Infectious diseases, for example tuberculosis, can cause a person or animal infected with the disease to produce antibodies. Identification of these antibodies in a sample taken from an infected individual can lead to a diagnosis of the disease.

US 2003/0219833 describes assays for detection of one or more analytes in a sample.
US 2003/0143652 relates to a method and kit for detecting the presence of at least one predesignated, target antibody to an mycobacterium in a sample selected from one or more patient bodily fluids. US 20070054335 relates to a universal test apparatus for detecting tuberculosis in non-primate mammals. WO 2010/086667 relates to a method of preparing a compound including mycolic acid residues and the use of the compound in detecting a pathogen.
WO 2012/153111 describes a method of determining the presence or absence of a biomarker in a sample. WO 2012/007903 describes a method of detecting markers for active tuberculosis in a serum sample. JP H07248329 describes a tubercular infectivity inspection membrane for detection of a tubercular infectivity related antibody.
WO 2012/131394 relates to a method of determining the presence or absence of a biomarker indicative of exposure to mycobacteria.

According to a first aspect of the present invention there is provided a method of determining the presence or absence in a sample of a biomarker, the method comprising:
(a) providing a porous substrate positioned within a housing, the porous substrate carrying an antigen selected from mycolic acids, derivatives and salts of mycolic acids and wax esters analogous to mycolic acids;
(b) contacting a surface of the substrate with the sample, the sample passing through the substrate to an opposite surface of the substrate;
(c) contacting the substrate with a composition comprising colloidal gold particles wherein the colloidal gold particles carry a secondary antibody; and
(d) observing the substrate, wherein when the biomarker is present in the sample a colour change in the region of the substrate which carries the antigen is observed; wherein the antigen is at least 90% pure; and
   wherein the substrate is positioned within the housing to enable the sample to pass through the substrate from one surface of the substrate to the opposite surface.

The first aspect of the invention relates to a method of detecting a biomarker. A biomarker as used herein refers to a molecule produced by a living organism which is characteristic of a particular condition. The biomarker may be a molecule which is produced when the organism has a particular disease and/or when the organism has been exposed to a pathogen. In some preferred embodiments the biomarker is a molecule which is produced when an organism has been infected with a pathogenic disease. Such a disease may be caused by infection, for example with bacteria, viruses or fungi.

In preferred embodiments the biomarker is an antibody. Preferably it is an antibody indicative of infection with a pathogenic disease.

In some embodiments the biomarker may be a molecule which is characteristic of a non-pathogenic condition, for example an auto-immune disease.

In preferred embodiments the present invention involves a method of determining the presence or absence of a biomarker indicative of exposure to mycobacteria or related species that produce analogues of mycolic acid, for example corynobacteria or rhodococcus.

In preferred embodiments, the present invention relates to a method of determining the presence or absence in a sample of a biomarker indicative of exposure to mycobacteria. By this we mean to refer to any molecule or combination of molecules that would be produced by an organism in response to exposure to mycobacteria. The organism may be a plant or an animal. Preferably it is an animal. Most preferably the sample donor is a mammal.

The method of the present invention may be used to determine the presence or absence of a biomarker indicative of exposure to non-pathogenic or environmental mycobacteria. However in preferred embodiments the biomarker is indicative of infection with a mycobacterial disease. In such embodiments the biomarker is an antibody indicative of infection with a mycobacterial disease.

The present invention preferably relates to a method of determining the presence or absence in a sample of an antibody indicative of infection with or exposure to mycobacteria.

The present invention may be used to determine the presence or absence of a disease antibody indicative of infection with any disease caused by infection with mycobacteria. Examples of such diseases include tuberculosis, leprosy, pulmonary disease, burili ulcer and bovine tuberculosis.

In preferred embodiments the method of the present invention is used to determine the presence or absence of an antibody indicative of infection with *Mycobacterium tuberculosis* and/or *Mycobacterium avium paratuberculosis.*

The present invention may also be used to determine the presence or absence of antibodies to lipids generated by exposure to non-pathogenic mycobacteria.

The invention finds particular utility in determining the presence or absence in a sample of disease antibodies indicative of the presence of tuberculosis. The sample may be taken from any animal suspected of infection with tuberculosis. Suitably the animal is a human. Suitably the animal is a bovine animal.

Step (a) of the method of the first aspect of the present invention involves providing a porous substrate which carries an antigen.

The porous substrate may be any material which allows another medium to pass through it. Suitably the porous substrate allows liquid compositions and semi-solid or viscous liquid compositions (for example gels and pastes) to pass through.

Any suitable porous substrate may be used. Suitably the porous substrate is a woven material. Preferably the porous substrate is a cellulosic material.

The porous material carries an antigen. The antigen may be carried within the porous material or on the surface of the porous surface.

Preferably the antigen forms a chemical interaction with the surface of the substrate. This may involve a polar interaction, for example dipole-dipole interactions or hydrogen bonding; or a non-polar interaction, for example Van der Waals forces.

In some preferred embodiments the antigen forms hydrogen bonds with functional groups at the surface of the substrate.

The antigen is selected from mycolic acids, wax esters analogous to mycolic acids, derivatives and salts thereof.

Such compounds are antigens for lipid antibodies generated by infection with mycobacteria.

The antigen may suitably be selected from one or more of the following classes of compounds:
(i) mycolic acids obtained from natural sources;
(ii) synthetically prepared mycolic acids;
(iii) salts of mycolic acids;
(iv) esters of mycolic acids (i) and/or (ii);
(v) sulfur-containing mycolic acids and/or salts or esters thereof;
(vi) simple structural analogues of mycolic acids and/or salts or esters thereof; and
(vii) mycolic acid wax esters and/or salts or esters thereof.

Mycolic acids obtained from natural sources (i) are typically available as mixtures. These typically contain different classes of mycolic acids and each class will usually contain a complex mixture of different homologues.

It is highly advantageous to use synthetically prepared mycolic acids (ii) since these are available as single compounds in high purity (for example greater than 95% or greater than 99%). The use of single compounds allows greater substrate selectivity to be achieved.

Salts of natural mycolic acids and/or synthetic mycolic acids (iii) may be useful. Suitable salts include ammonium, alkali metal and alkaline earth metal salts, for example salts of lithium, potassium, sodium, calcium or barium.

Suitable esters (iv) for use as antigens include esters of simple alcohols and sugar esters. Sugar esters are particularly preferred. Some preferred sugar esters of mycolic acids are trehalose monomycolates or trehalose dimycolates (also known as cord factors). Cord factors can be isolated as complex mixtures from natural sources. Esters of mycolic acids for use herein as antigens may be synthetically prepared. They may be prepared by esterification of synthetically prepared mycolic acids or by esterification of mycolic acids isolated from natural sources.

By sulfur-containing mycolic acids and/or esters or salts thereof (v) we mean to refer to synthetic compounds which are analogues of natural mycolic acid compounds rather than naturally occuring compounds that contain sulfur. Suitable sulfur-containing mycolic acid derivatives may include any compound in which one or more carbon atoms and/or one or more oxygen atoms of a mycolic acid derived compound have been replaced by a sulfur atom. Sulfur-containing mycolic acid derivatives also include compounds in which a hydrogen substituent has been replaced with a moiety "SX" wherein X is hydrogen, SR¹ or COR² in which R¹ is an optionally substituted alkyl, alkenyl, acyl or aryl group and R² is an optionally substituted alkyl, alkenyl or aryl group.

Simple structural analogues of mycolic acids and/or esters or salts thereof (vi) which may be used herein as antigens include compounds which include fewer functional groups and/or stereocentres than are found in natural mycolic acid compounds but have many structural features in common, for example they include a similar number of carbon atoms and have a simpler substitution pattern.

Mycolic acid wax esters (vii) include a cyclopropyl group or an alkene and an internal ester group. These can be isolated from natural sources (typically as mixtures of homologues) or they can be prepared synthetically. Salts and esters of these wax esters thereof can also be used.

Preferably the antigen is selected from mycolic acids, and salts and esters thereof. Preferably the antigen is a synthetically prepared antigen.

Suitably the antigen is selected from mycolic acids and salts and esters thereof. Preferably the antigen is a synthetically prepared antigen.

Suitably the antigen is selected from mycolic acids and esters thereof. Preferably the antigen is a synthetically prepared antigen. When the antigen is an ester preferably the mycolic acid residue or residues present in the ester are derived from synthetically prepared mycolic acids.

Suitable compounds for use herein as antigens include the mycolic acid compounds described in WO2009/130506, WO2009/130508 and the sugar esters described in WO 2010/086667.

Mycolic acids are long chain fatty acid compounds typically having 60 to 90 carbon atoms and are found in the cell walls of mycobacteria.

As is shown by way of example in formula I, two moieties can be distinguished in each mycolic acid: the main branch, or meromycolate moiety, and the mycolic motif, an α-alkyl β-hydroxy acid. The structure of the mycolic motif is common to each naturally occurring mycolic acid, except for minor variations in the length of the chain in the α-position. The two stereocentres in the α and β positions relative to the carboxylic group present in all natural mycolic acids have, when examined, always been found to both be in the (*R*)-configuration in these natural products. On the other hand, the meromycolate section, which generally contains two functionalities and three long chains (a, b, c in formula I), can be differently substituted in both the proximal (the one nearer the hydroxy-acid) and the distal position (further from the carboxylic acid).

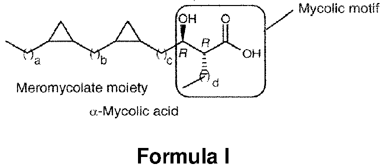

The mycolic acids are broadly separated into classes, according to the groups present in the meromycolate moiety. The proximal or distal functional groups can include cyclopropanes, double bonds, an epoxy group, a methoxy group, carbonyl group, carboxyl group or methyl group.

Suitable mycolic acid classes for use herein as antigens include keto mycolic acids having the structure shown in formula IIa; hydroxy mycolic acids having the structure shown in formula IIb; alpha mycolic acids having the structure shown in formula IIc; and methoxy mycolic acids having the structure shown in formula IId.

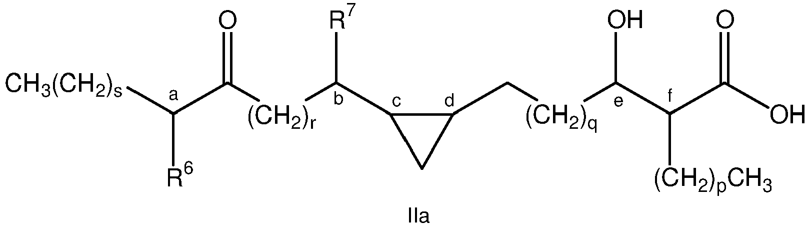

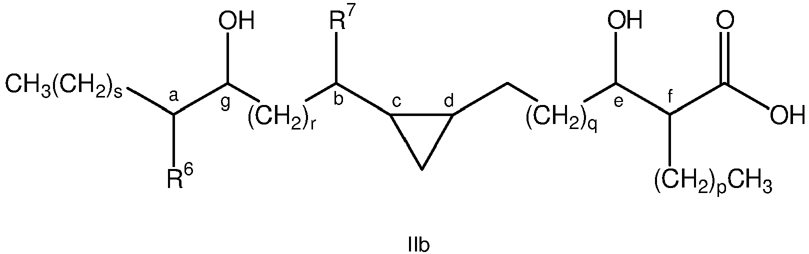

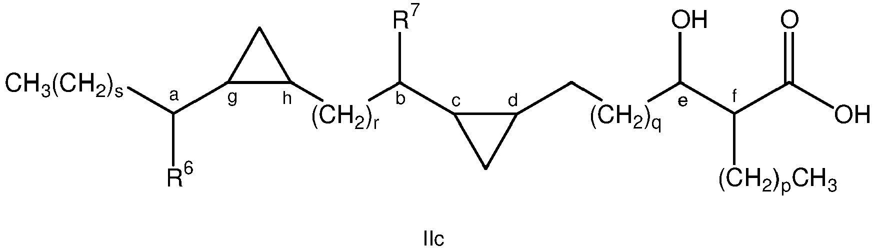

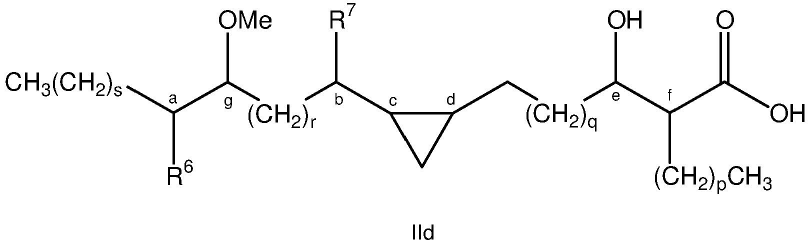

In each of the structures IIa, IIb, IIc and IId R⁶ may be hydrogen or C₁ to C₄ alkyl. Preferably R⁶ is hydrogen or methyl.

In each of the structures IIa, IIb, IIc and IId R⁷ may be hydrogen or C₁ to C₄ alkyl. Preferably R⁷ is hydrogen or methyl.

In each of the structures IIa, IIb, IIc and IId p is preferably from 4 to 40, preferably from 8 to 36, more preferably from 12 to 32, for example from 16 to 30, more preferably from 20 to 28, for example from 22 to 26.

In the structures IIa, IIb, IIc and IId q is preferably from 2 to 40, more preferably from 4 to 36, for example from 6 to 30, preferably from 8 to 24, for example from 10 to 20 and preferably from 12 to 18.

In the structures IIa, IIb, IIc and IId, r is preferably from 2 to 40, for example from 6 to 36, preferably from 10 to 32, for example from 12 to 28, and preferably from 14 to 24.

In the structures IIa, IIb, IIc and IId, s is preferably from 2 to 40, for example from 6 to 36, preferably from 10 to 32, for example from 12 to 28, and preferably from 14 to 24.

In the structures IIa, IIb, IIc and IId, each of the chiral centres indicated at a, b, c, d, e, f, g and h may independently have either an (R) or an (S) configuration. Each cyclopropyl group may have either absolute stereochemistry and may have a trans or a cis configuration.

Any of the stereocentres indicated by a, b, c, d, e, f, g or h may be racemic. In the case of structure IIa it is possible that the stereocentre designated a will be racemic as this is a readily epimerisable position.

In addition to the compounds illustrated by the structures IIa, IIb, IIc and IId, other classes of mycolic acids may be useful as antigens in the present invention. Suitable mycolic acid compounds may include an alkene functional group instead of the proximal cyclopropyl group shown on figures IIa, IIb, IIc and IId. Further suitable classes of mycolic acid include those substituted with epoxy and alkene groups in the meromycolate moiety in place of the distal cyclopropyl, methoxy, hydroxy or keto group. The proximal groups in such compounds may be alkene or cyclopropyl. The structure of such compounds will be known to the person skilled in the art.

The antigen used in the method of the present invention is preferably a mycolic acid-derived antigen selected from keto mycolic acids, hydroxy mycolic acids, alpha mycolic acids, methoxy mycolic acids, epoxy mycolic acids and alkene mycolic acids.

Each of the above-described mycolic acid compounds may be used as single compounds prepared synthetically and/or may be included in mixtures of synthetic compounds and/or may be included in mixtures isolated from natural sources. Any of these compounds could be used in the preparation of synthetic esters or be present in naturally occurring cord factors.

In some embodiments the antigen is an ester of a mycolic acid.

Suitable esters include esters of monohydric alcohols, polyhydric alcohols and sugars.

Ester antigens for use herein may be monoesters, diesters or polyesters. Each ester may include one or more mycolic acid groups and one or more alcohol or sugar moieties. Antigens which are mixed esters including alcohols and sugars may also be used, for example compounds including an alcohol ester moiety and a sugar ester moiety.

In some preferred embodiments the antigen is a sugar ester of a mycolic acid.

When a sugar ester is present this may be a monosaccharide, disaccharide or an oligosaccharide.

Suitable sugar units which may be included are those based on hexoses and those based on pentoses.

Some especially preferred sugar esters for use herein are glucose esters.

Other especially preferred sugar esters are trehalose esters. Suitable trehalose esters include trehalose monomycolates and trehalose dimycolates. Trehalose dimycolates (or cord factors) have the structure shown in formula IV wherein MA represents the residue of a mycolic acid:

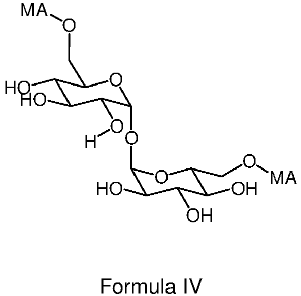

In formula IV each MA residue may be of the same or a different mycolic acid.

Suitable sugar ester compounds include monomycolates, dimycolates, trimycolates and tetramycolates; and mixed esters of sugar and alcohols.

In some embodiments the antigen comprises a keto mycolic acid or a derivative thereof.

In some embodiments the antigen comprises a hydroxy mycolic acid or a derivative thereof.

In some embodiments the antigen comprises a methoxy mycolic acid or a derivative thereof.

In some embodiments the antigen comprises an alpha mycolic acid or a derivative thereof.

In some embodiments the antigen comprises an epoxy mycolic acid or a derivative thereof

In some embodiments the antigen comprises an alkene mycolic acid or a derivative thereof.

Simple structural analogues of mycolic acids which can be used herein as antigens include compounds having the structures indicated in formula IIa, IIb, IIc or IId in which some or all of the stereocentres a, b, c, d, e, f, g and h are racemic and in which R⁶ and R⁷ may each be hydrogen.

Simple structural analogues of cord factors include compounds having the structure indicated in formula IV wherein one or each MA group is a simple analogue as described above or in which one or each MA is a fatty acid having a long carbon chain.

In some embodiments the antigen may be a mycolic acid wax ester. These compounds suitably have the formula (V): 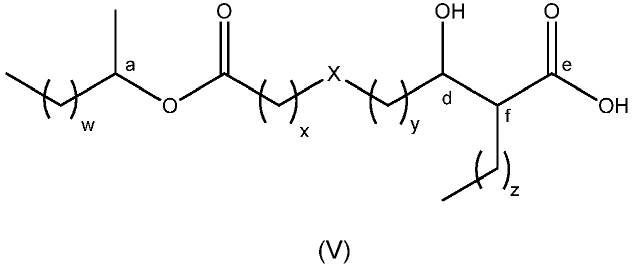 wherein w is from 2 to 40, x is from 2 to 40, y is from 2 to 40, z is from 4 to 40 and X is a three carbon fragment including an alkane, alkene or cyclopropyl moiety.

Suitably X is a group of formula (VIa), (VIb), (VIc) or (VId) : 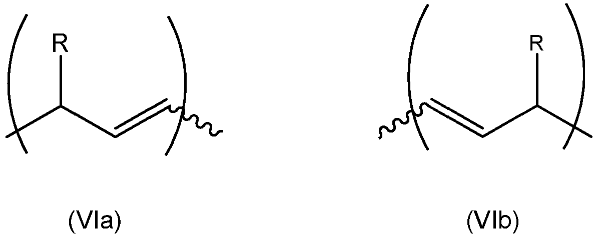 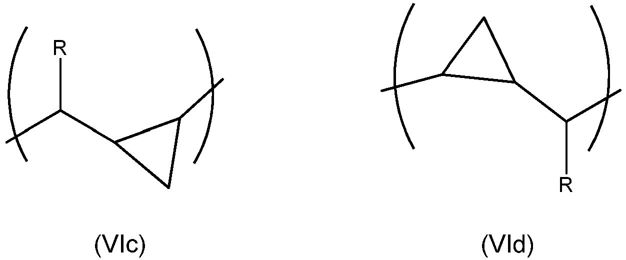 wherein R is methyl or hydrogen. The double bond in formula (Via) and (Vlb) may be cis or trans.

In embodiments in which X is (Via) or (Vlb), when the double bond is trans, R is preferably methyl. When the double bond is cis, R is preferably H.

The cyclopropyl group of fragment (Vlc) or (Vld) may be cis or trans.

In preferred embodiments in which X is (VIc) or (VId), R is preferably methyl.

In some especially preferred embodiments X is a fragment of formula (VIc) and the wax ester has the formula (VII):

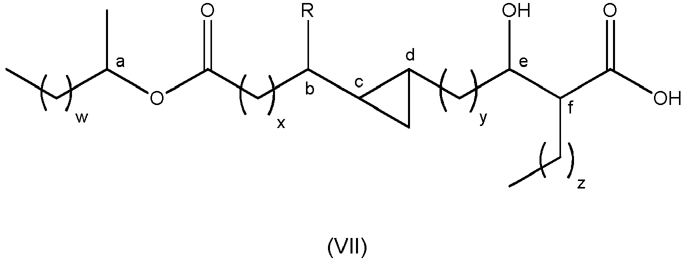

Preferably w is from 10 to 32, preferably from 4 to 24; x is from 8 to 24, preferably from 12 to 18; y is from 10 to 32, preferably from 14 to 24; z is from 16 to 30; preferably from 22 to 26; and R is C₁ to C₄ alkyl, preferably methyl.

Esters of wax esters, for example sugar esters may also be used as antigens. The structures of the sugar esters are analogous to the sugar esters of the mycolic acids described above.

In preferred embodiments the substrate carries a synthetic antigen.

Preferably the substrate carries a synthetic mycolic acid or an ester of a sythetic mycolic acid.

The substrate may suitably carry an antigen which is at least 95% pure or at least 99% pure.

Suitably the substrate carries a synthetic antigen which is at least 95% or at least 99% pure.

In some embodiments the substrate may carry a mixture of two or more antigens. In preferred embodiments in which mixtures are present the structure of all compounds and preferably the relative amounts of each compound are known.

Preferred mixtures are mixtures of synthetically prepared antigens.

An advantage of using synthetically prepared antigens is that the compounds may be provided in high purity. Natural mycolic acids contain complex mixtures of different classes of mycolic acids and different homologues which are very difficult to separate. The use of synthetic compounds allows single compounds or known mixtures to be used. This enables antigens having a high degree of specificity and/or sensitivity for a particular antibody or antibodies to be used.

Step (a) involves providing a substrate which carries an antigen.

To prepare the substrate the antigen may be directly applied to the substrate.

In some preferred embodiments in which the substrate is a cellulose material and the antigen is a mycolic acid or a salt or ester thereof, a solution or suspension of the antigen may be applied to the substrate and the solvent allowed to evaporate. Without wishing to be bound by theory it is believed that hydrogen bonds form between the mycolic motif and/or residues in the sugar or glycerol residue of an ester and hydroxy groups of the cellulose.

Suitably the antigen is dissolved in a solvent. This may be an organic solvent, for example a mixture of hexanes; or an aqueous solvent, for example a buffer. The solution or suspension of antigen is suitably applied to the substrate and the solvent is then allowed to evaporate.

The antigen may be encapsulated, for example in a liposome.

Suitably a small spot of antigen is applied to the substrate.

In some embodiments more than one antigen may be applied to the substrate. Different antigens may be applied to the same area of the substrate. In some embodiments different antigens may be applied to different areas of the substrate.

Areas of the substrate which do not contain an antigen spot or spots may be "blocked", for example an impermeable coating may be applied to the surface of the substrate in these regions.

In some preferred embodiments the substrate carries at least two different mycolic-acid derived antigens at different positions. In some embodiments the substrate may carry more than two mycolic-acid derived antigens at different positions. It may suitably carry at least 3 different mycolic acid derived antigens at different positions, for example at least 4, at least 5 or at least 6.

In some embodiments the substrate carries from 5 to 8 different antigens at different positions. Preferably each of these antigens is synthetically prepared. Preferably each is at least 95% pure, for example at least 99% pure. The use of a combination of a number of different antigens allows a higher degree of sensitivity and specificity to be achieved and enables distinction between different mycobacterial diseases.

In step (b) of the method of the present invention the substrate is contacted with the sample.

Any suitable sample may be tested using the present invention. Suitably the sample is selected from serum, blood, saliva, urine or sputum. In some embodiments the sample is blood. It may be serum. It may be whole blood.

When the substrate is contacted with a sample which contains a biomarker this biomarker becomes bound to or interacts with the antigen carried on the substrate.

The sample may be directly contacted with the substrate or it may be diluted, filtered or otherwise purified prior to contact with the substrate. Suitable diluents, filtration methods and purification techniques will be known to the person skilled in the art.

The sample is suitably contacted with the substrate as a liquid or semi-liquid composition. Preferably it is a liquid composition.

In some embodiments the sample is diluted before contacting with the substrate. It may be diluted with an aqueous composition, suitably an aqueous buffer. Preferably it is diluted with an aqueous buffer having a pH of 6 to 8, preferably about 7. A casein buffer is especially preferred.

In some embodiments the substrate may be immersed in the sample or a composition comprising the sample.

In some embodiments the sample or a composition comprising the sample may be passed over the surface of the substrate.

The sample or a composition comprising the sample is contacted with a surface of the substrate and allowed to pass through the substrate.

The substrate is suitably a sheet material. The sample or a composition comprising the sample may pass from one edge of the substrate to the opposite edge or may be contacted with a face of the substrate and pass through the substrate to the opposite face.

The sample or a composition comprising the sample may be contacted with the entire area of the substrate or a portion of the substrate, suitably the portion which carries the antigen.

Step (c) involves contacting the substrate with a composition comprising a secondary antibody.

Step (c) involves contacting the substrate with a composition comprising colloidal gold particles wherein the colloidal gold particles carry a secondary antibody.

The composition comprising colloidal gold particles is preferably an aqueous suspension of gold nanoparticles.

Suitably the nanoparticles have an average size of from 1 to 200nm, preferably from 5 to 150nm, suitably from 10 to 100nm, suitably from 20 to 80nm, for example about 40nm.

The composition may comprise one or more further ingredients for example cosolvents, preservatives, or buffering agents.

Preferably the composition comprises a buffer. Preferably the composition has a pH of from 5 to 9, preferably from 6 to 8, for example about 7. In some especially preferred embodiments the composition comprises a casein buffer.

Suitably the nanoparticles of gold carry a secondary antibody on their surface.

The antibody suitably forms an interaction with the surface of the gold nanoparticles.

In some preferred embodiments the gold nanoparticles are coated with a composition which promotes interaction with the secondary antibody. Preferably the gold particles are coated with a polymer. Suitable polymers are able to stabilize the gold particles and covalently bind antibodies.

The method of the present invention is used to determine the presence or absence of a biomarker in a sample.

Any antibody or antibody conjugate which interacts with the biomarker may be used as the secondary antibody. Preferred secondary antibodies include Immunoglobulin G and Immunoglobulin M.

Preferably after step (c) in which the substrate is contacted with a composition comprising a secondary antibody, the substrate is suitably washed. Preferably it is washed with a composition comprising a buffer. Preferably it is washed with a composition of pH 6 to 8, suitably about 7. An aqueous composition comprising a casein buffer is especially preferred.

Step (d) involves observing the substrate.

Suitably in embodiments in which the biomarker is present in the sample a colour change in the region of the substrate which carries the antigen is observed. If the biomarker is absent no colour change is observed.

Thus in preferred embodiments a positive sample causes a colour change and a negative sample causes no colour change.

In some embodiments step (d) may involve quantitatively measuring the colour change. Quantitative analysis of this type may also help determine the severity of infection with a mycobacterial disease.

In some embodiments the colour change can be measured quantitatively. This can be carried out by a portable device, for example a mobile telephone. The quantitative data can be stored electronically and analysed. Statistical analysis may be performed on multiple measurements and comparisons made with earlier results to determine the likelihood that an individual is infected with a particular disease. In embodiments in which the substrate carries multiple antigens at different positions, the colour change can be measured at each position and compared. Multiple readings help improve the specificity and sensitivity of the results.

Step (d) may also involve measuring the colour change over time. This information may also be useful in determining the type or extent of infection with a mycobacterial disease.

However in preferred embodiments step (d) may involve simply visually observing the presence or absence of a colour change to provide a qualitative assessment.

In the method of the present invention when the sample is contacted with the substrate in step (b), if the biomarker is present it interacts with the antigen carried on the substrate and is thus "tethered" to the surface of the substrate.

If no biomarker is present no interaction occurs with the antigen and the biomarker is not present at the surface of the substrate.

In step (c) the substrate is contacted with a composition comprising colloidal gold particles which carries a secondary antibody. If following step (b) the biomarker is carried on the surface of the substrate the secondary antibody interacts with the biomarker and tethers the gold particles to the substrate. If no biomarker is carried on the surface of the substrate then the gold particles pass through the substrate.

The gold particles have a red colour. Thus when a biomarker is present the region of the substrate which carries the antigen is red at the end of step (c). If no biomarker is present no colouration of the substrate is observed.

A particular advantage of the present invention is that it enables a very quick, simple test to be carried out to determine whether or not a particular sample contains a biomarker. Suitably it is used to determine whether or not the sample contains a biomarker indicative of exposure to mycobacteria, for example an antibody indicative of infection with or exposure to a mycobacterial disease. The method of the present invention may be carried out at remote locations. The colour change provides an immediate indication whether the provider of the sample is infected with a mycobacterial disease.

According to a second aspect of the present invention there is provided a kit for determining the presence or absence of a biomarker in a sample, the kit comprising:
(i) a porous substrate positioned within a housing, the porous substrate carrying an antigen which is at least 90% pure and which is selected from mycolic acids, derivatives and salts of mycolic acids and wax esters analogous to mycolic acids; and
(ii) a composition comprising colloidal gold particles wherein the colloidal gold particles carry a secondary antibody;
wherein the substrate is positioned within the housing to enable the sample to pass through the substrate from one surface of the substrate to an opposite surface of the substrate.

In the kit of the second aspect the substrate is located within a suitable housing.

The substrate is positioned within the housing so as to enable the sample to contact the substrate.

Suitably the housing includes an aperture to enable the sample to contact the substrate in the region which carries the antigen.

The substrate is positioned within the housing to enable the sample to pass through from one side of the substrate to the other.

The housing may further comprise a chamber to collect the sample and other compositions after they pass through the substrate. The chamber may include an absorbent material to soak up the excess sample, excess gold composition and/or any washing compositions.

The housing may be made from any suitable material. Preferably it is a plastic housing.

The absorbent material is preferably a sponge-like material.

The present invention may relate to a device comprising a housing and a substrate; wherein the substrate carries an antigen and is located within the housing; and wherein the antigen is selected from mycolic acids, wax esters analogous to mycolic acids and salts and esters thereof.

The kit of the second aspect preferably comprises such a device and a composition comprising colloidal gold particles which carry a secondary antibody on their surface.

When used to analyse known samples of sera from individuals some of whom had been infected with *M*. *tuberculosis*, the method of the present invention was found to provide a faster method of discrimination between positive and negative samples compared with using standard methods based on ELISA assays. The method of the present invention is also more suitable for point of care use than ELISA, for example in environments with limited access to hospitals and laboratories.

A further advantage of the device of the present invention is that a single device may be provided for each individual person. The antigens can be selected accordingly. Thus the device is useful in personalised medicine.

When the present invention is used to test for disease antibodies indicative of infection with a mycobacterial disease it can provide results very quickly, with good accuracy and at relatively low cost. It therefore provides significant advantages over the prior art.

The invention will now be further described with reference to Figures 1 to 4.
Figure 1 shows a cross-sectional view of a device used in the present invention;
Figure 2 shows a schematic top view of the device of Figure 1;
Figure 3 shows a cross-sectional view of a further device of the present invention; and
Figure 4 shows a schematic top view of the device of Figure 3.

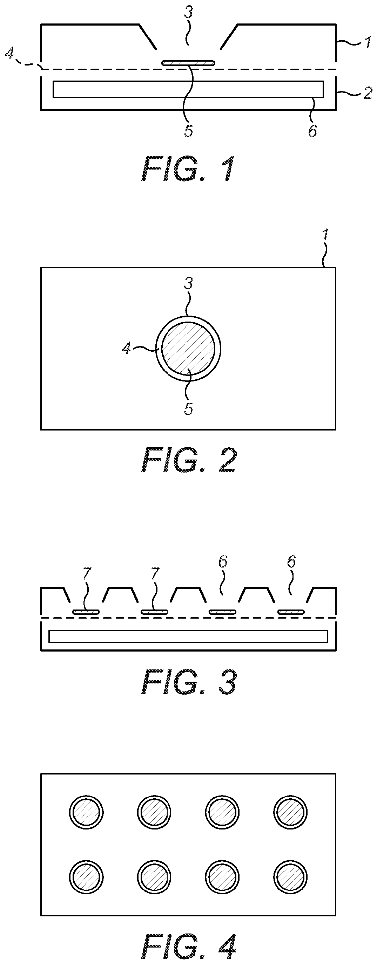

The device of Figures 1 and 2 comprises a plastic housing having an upper portion 1 and a lower portion 2. The upper portion includes an aperture 3. The sides of the aperture are shaped to direct liquid through the aperture. A porous substrate 4 is located within the housing. An antigen is provided on the substrate in the region below the aperture 5.

The device of Figures 3 and 4 includes an array of apertures 6 and an array of spots of antigens 7. This device may be used to test a plurality of samples using the same antigen and/or to test the same sample using a plurality of antigens.

The invention will now be further described with reference to the following non-limiting examples.

### Example 1 - general procedure

An antigen solution (2mg/ml in hexane) was applied to provide a series of spots on a nitrocellulose membrane.

The antigen had the following structure:

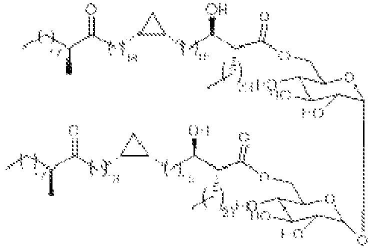

1 ml of a serum sample (diluted to 50% with a casein/PBS buffer) was applied to each spot and allowed to run through the membrane. The membrane was then washed with distilled water.

1ml of a colloidal gold suspension was applied to the membrane and allowed to flow through. The membrane was again washed. The gold particles were coated with a polymer and had a secondary antibody (IgG/IgM) attached.

Testing was carried out using two serum samples know to be positive for TB (chips 1 and 3) and two known to be negative for TB (chips 2 and 4). A control sample was also tested.

On chips 1, 2 and the control chip, amounts of antigen were applied as follows:

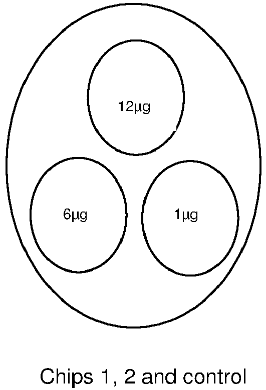

On chips 3 and 4 the following amounts of antigen were applied:

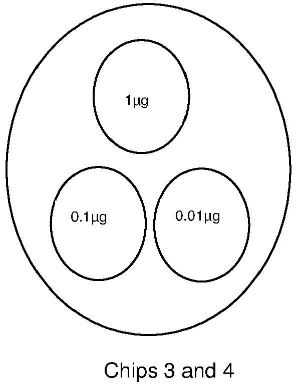

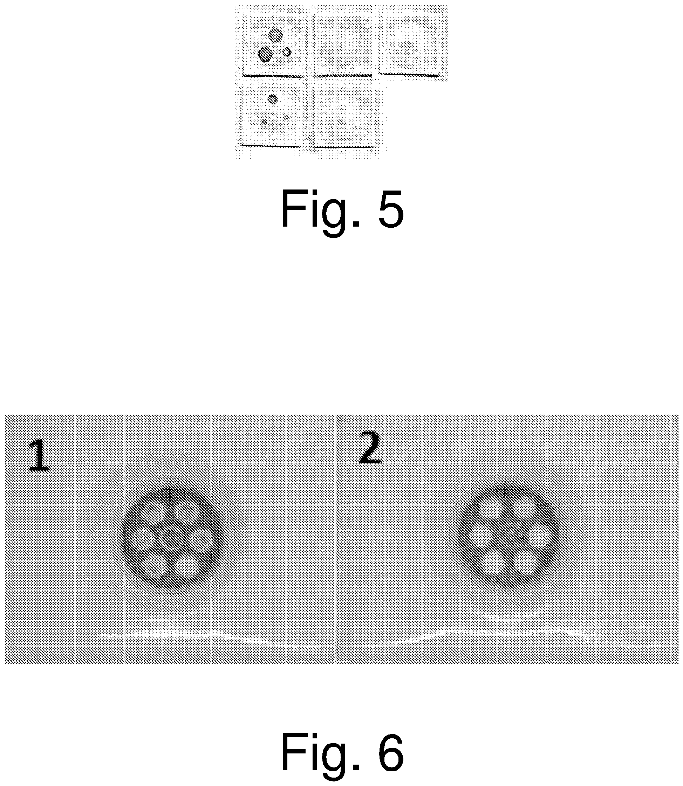

The results are shown in figure 5.

Chips 1 and 2 and the control are shown left to right across the top and chips 3 and 4 are left to right on the bottom row.

These results clearly show that the present invention provides a quick method to distinguish between serum examples which are positive and negative for TB.

It is also possible to measure the colour quantitatively using digital techniques.

### Example 2

A wax template was printed onto a nitrocellulose membrane leaving six blank spots around a central spot.

1µg of the antigen of Example 1 (1mg/ml solution) was applied to five of the blank spots. IgG serum (1µl, 0.05mg/ml solution) was applied to the central spot. The final spot (bottom right) was left blank.

A serum sample known to be positive for tuberculosis has passed through a first device and a serum sample known to be negative for tuberculosis has passed through a second device.

In each case 300µl of each of the following solutions was added to the device and allowed to flow through:
1. the serum sample diluted in a 1:50 ratio by volume with a composition comprising 0.5% (w/v) casein, 0.1% (v/vs) HMPC (hypromellose), 0.1% (v/v) tween 20 and adjusted to pH 7.4 with phosphate buffered saline (PBS);
2. distilled water;
3. a solution of gold nanoparticles which carry Immunoglobulin G (IgG) diluted to an optical density of 1 in a composition comprising 0.5% (w/v) casein, 0.1% (v/vs) HMPC (hypromellose), 0.1% (v/v) tween 20 and adjusted to pH 7.4 with phosphate buffered saline (PBS);
4. distilled water.

Figure 6 shows a photograph of the two devices at the end of the test. Device 1 had the positive serum and it can be seen that the positive control is in the centre and there is one negative control. The other spots have all changed colour.

For device 2 only the positive control has changed colour.

A photograph of the device was taken using a mobile phone and an application recorded the average RGB pixel count per spot.

The number is then subtracted from 255. The test score is then divided by the control spot, and interpreted based on the following criteria:
Positive when t/+ c > 1 and t/- c > 1. Negative when t/- c < 1 and t/+ c < 1.

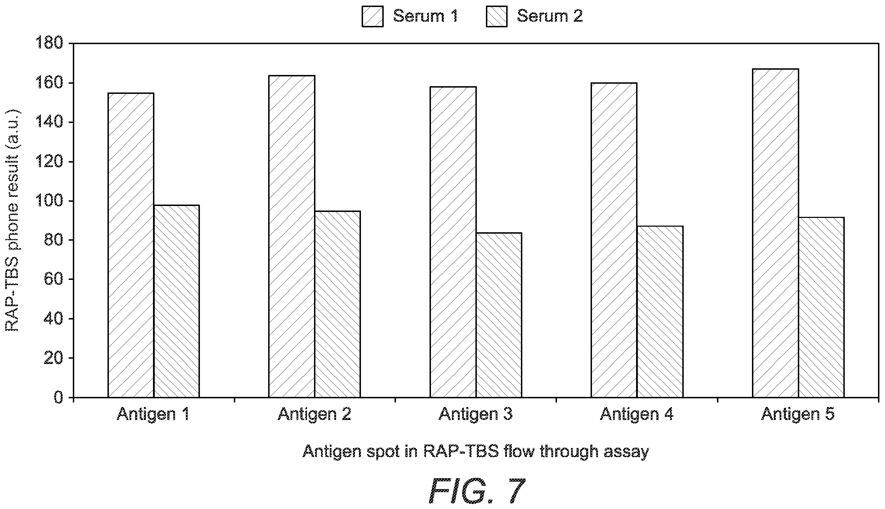

Figure 7 shows the phone results for each antigen spot graphically.

ELISA tests were carried out on the same samples using the same antigen as a comparison.

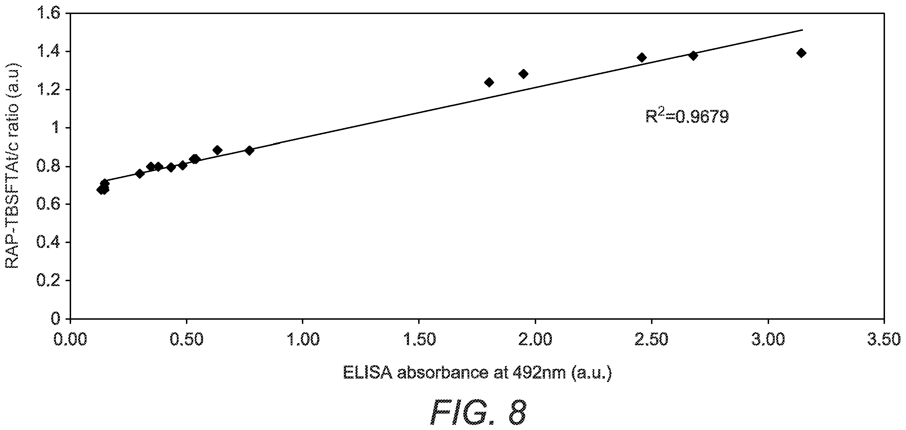

Figure 8 shows that the correlation of the mobile phone data of Figure 7 with the ELISA results for a range of serum samples.

## Claims

1. A method of determining the presence or absence in a sample of a biomarker, the method comprising:
(a) providing a porous substrate positioned within a housing, the porous substrate carrying an antigen selected from mycolic acids, derivatives and salts of mycolic acids and wax esters analogous to mycolic acids;
(b) contacting a surface of the substrate with the sample, the sample passing through the substrate to an opposite surface of the substrate;
(c) contacting the substrate with a composition comprising colloidal gold particles wherein the colloidal gold particles carry a secondary antibody; and
(d) observing the substrate, wherein when the biomarker is present in the sample a colour change in the region of the substrate which carries the antigen is observed; wherein the antigen is at least 90% pure; and
wherein the substrate is positioned within the housing to enable the sample to pass through the substrate from one surface of the substrate to the opposite surface.

2. The method according to claim 1, wherein the composition comprising colloidal gold particles is an aqueous suspension of gold nanoparticles.

3. The method according to any preceding claim which is a method of determining the presence or absence in a sample of an antibody indicative of infection with or exposure to mycobacteria.

4. The method according any preceding claim, wherein the porous substrate is a cellulosic material.

5. The method according to any preceding claim wherein the antigen is selected from one or more of the following classes of compounds:
(i) mycolic acids obtained from natural sources;
(ii) synthetically prepared mycolic acids;
(iii) salts of mycolic acids;
(iv) esters of mycolic acids (i) and/or (ii);
(v) sulfur-containing mycolic acids and/or salts or esters thereof;
(vi) simple structural analogues of mycolic acids and/or salts or esters thereof; and
(vii) mycolic acid wax esters and/or salts or esters thereof.

6. The method according to any preceding claim wherein the substrate carries a synthetic antigen.

7. The method according to any preceding claim in which the sample is selected from serum, blood, saliva, urine or sputum.

8. The method according to any preceding claim in which step (d) involves visually observing the presence or absence of a colour change.

9. A kit for determining the presence or absence of a biomarker in a sample, the kit comprising:
(i) a porous substrate positioned within a housing, the porous substrate carrying an antigen which is at least 90% pure and which is selected from mycolic acids, derivatives and salts of mycolic acids and wax esters analogous to mycolic acids; and
(ii) a composition comprising colloidal gold particles wherein the colloidal gold particles carry a secondary antibody;
wherein the substrate is positioned within the housing to enable the sample to pass through the substrate from one surface of the substrate to an opposite surface of the substrate.

## Patentansprüche

1. Verfahren zur Feststellung des Vorhandenseins oder Fehlens eines Biomarkers in einer Probe, wobei das Verfahren Folgendes umfasst:
(a) Bereitstellen eines porösen, sich in einem Gehäuse befindlichen Substrats, wobei das poröse Substrat ein Antigen, das aus Mykolsäuren, Derivaten und Salzen von Mykolsäuren und zu Mykolsäuren analogen Wachsestern ausgewählt ist, trägt,
(b) In-Kontakt-Bringen einer Oberfläche des Substrats mit der Probe, wobei die Probe das Substrat zu einer gegenüberliegenden Oberfläche des Substrats durchläuft,
(c) In-Kontakt-Bringen des Substrats mit einer kolloidale Goldpartikel umfassenden Zusammensetzung, wobei die kolloidalen Goldpartikel einen sekundären Antikörper tragen, und
(d) Beobachten des Substrats, wobei, wenn der Biomarker in der Probe vorhanden ist, eine Farbänderung in der Umgebung des das Antigen tragenden Substrats beobachtet wird,
wobei das Antigen zu mindestens 90 % rein ist und wobei sich das Substrat innerhalb des Gehäuses befindet, damit die Probe das Substrat von einer Oberfläche des Substrats zu der gegenüberliegenden Oberfläche durchlaufen kann.

2. Verfahren nach Anspruch 1, wobei es sich bei der kolloidale Goldpartikel umfassenden Zusammensetzung um eine wässrige Suspension von Goldnanopartikeln handelt.

3. Verfahren nach einem der vorhergehenden Ansprüche, bei dem es sich um ein Verfahren zur Feststellung des Vorhandenseins oder Fehlens eines Antikörpers, der auf eine Infektion mit oder einen Kontakt zu Mykobakterien hinweist, handelt.

4. Verfahren nach einem der vorhergehenden Ansprüche, wobei es sich bei dem porösen Substrat um ein cellulosisches Material handelt.

5. Verfahren nach einem der vorhergehenden Ansprüche, wobei das Antigen aus einer oder mehreren der folgenden Klassen von Verbindungen ausgewählt ist:
(i) Mykolsäuren, die aus natürlichen Quellen erhalten wurden,
(ii) synthetisch hergestellten Mykolsäuren,
(iii) Salzen von Mykolsäuren,
(iv) Estern von Mykolsäuren (i) und/oder (ii),
(v) schwefelhaltigen Mykolsäuren und/oder Salzen oder Estern davon,
(vi) einfachen Strukturanaloga von Mykolsäuren und/oder Salzen oder Estern davon und
(vii) Mykolsäurewachsestern und/oder Salzen oder Estern davon.

6. Verfahren nach einem der vorhergehenden Ansprüche, wobei das Substrat ein synthetisches Antigen trägt.

7. Verfahren nach einem der vorhergehenden Ansprüche, bei dem die Probe aus Serum, Blut, Speichel, Urin oder Sputum ausgewählt ist.

8. Verfahren nach einem der vorhergehenden Ansprüche, bei dem Schritt (d) das visuelle Beobachten des Vorhandenseins oder Fehlens einer Farbänderung beinhaltet.

9. Kit zur Feststellung des Vorhandenseins oder Fehlens eines Biomarkers in einer Probe, wobei das Kit Folgendes umfasst:
(i) ein poröses, sich in einem Gehäuse befindliches Substrat, wobei das poröse Substrat ein Antigen, das zu mindestens 90 % rein ist und das aus Mykolsäuren, Derivaten und Salzen von Mykolsäuren und aus zu Mykolsäuren analogen Wachsestern ausgewählt ist, trägt, und
(ii) eine kolloidale Goldpartikel umfassende Zusammensetzung, wobei die kolloidalen Goldpartikel einen sekundären Antikörper tragen,
wobei sich das Substrat innerhalb des Gehäuses befindet, damit die Probe das Substrat von einer Oberfläche des Substrats zu einer gegenüberliegenden Oberfläche des Substrats durchlaufen kann.

## Revendications

1. Méthode de détermination de la présence ou de l'absence d'un biomarqueur dans un échantillon, la méthode comprenant :
(a) la mise à disposition d'un substrat poreux positionné au sein d'un boîtier, le substrat poreux portant un antigène choisi parmi les acides mycoliques, les dérivés et sels d'acides mycoliques et les esters de cire analogues des acides mycoliques ;
(b) la mise en contact d'une surface du substrat avec l'échantillon, l'échantillon passant à travers le substrat vers une surface opposée du substrat ;
(c) la mise en contact du substrat avec une composition comprenant des particules d'or colloïdal, où les particules d'or colloïdal portent un anticorps secondaire ; et
(d) l'observation du substrat, où lorsque le biomarqueur est présent dans l'échantillon, on observe un changement de la couleur dans la région du substrat qui porte l'antigène ;
où l'antigène présente une pureté d'au moins 90 % ; et où le substrat est positionné au sein du boîtier de façon à permettre à l'échantillon de passer à travers le substrat de l'une des surfaces du substrat vers la surface opposée.

2. Méthode selon la revendication 1, dans laquelle la composition comprend des particules d'or colloïdal est une suspension aqueuse de nanoparticules d'or.

3. Méthode selon l'une quelconque des revendications précédentes, qui est une méthode de détermination de la présence ou de l'absence, dans un échantillon, d'un anticorps constituant une indication d'une infection par, ou d'une exposition à, des mycobactéries.

4. Méthode selon l'une quelconque des revendications précédentes, dans laquelle le substrat poreux est un matériau cellulosique.

5. Méthode selon l'une quelconque des revendications précédentes, dans laquelle l'antigène est choisi parmi l'une ou plusieurs parmi les classes suivantes de composés :
(i) les acides mycoliques obtenus à partir de sources naturelles ;
(ii) les acides mycoliques préparés par voie synthétique ;
(iii) les sels d'acides mycoliques ;
(iv) les esters des acides mycoliques (i) et/ou (ii) ;
(v) les acides mycoliques soufrés et/ou les sels ou esters de ceux-ci ;
(vi) les analogues structurels simples d'acides mycoliques et/ou les sels ou esters de ceux-ci ; et
(vii) les esters de cire d'acides mycoliques et/ou les sels ou esters de ceux-ci.

6. Méthode selon l'une quelconque des revendications précédentes, dans laquelle le substrat porte un antigène synthétique.

7. Méthode selon l'une quelconque des revendications précédentes, dans laquelle l'échantillon est choisi parmi le sérum, le sang, la salive, l'urine ou les crachats.

8. Méthode selon l'une quelconque des revendications précédentes, dans laquelle l'étape (d) met en jeu l'observation visuelle de la présence ou de l'absence d'un changement de couleur.

9. Kit de détermination de la présence ou de l'absence d'un biomarqueur dans un échantillon, le kit comprenant :
(i) un substrat poreux positionné au sein d'un boîtier, le substrat poreux portant un antigène qui présente une pureté d'au moins 90 % et qui est choisi parmi les acides mycoliques, les dérivés et sels d'acides mycoliques et les esters de cire analogues des acides mycoliques ; et
(ii) une composition comprenant des particules d'or colloïdal, où les particules d'or colloïdal portent un anticorps secondaire ;
où le substrat est positionné au sein du boîtier de façon à permettre à l'échantillon de passer à travers le substrat de l'une des surfaces du substrat vers une surface opposée du substrat.
